# EUROPEAN PATENT SPECIFICATION

(11) **EP 3 317 794 B1**
(45) Date of publication and mention of the grant of the patent: **07.08.2019**
(21) Application number: 16741365.7
(22) Date of filing: 30.06.2016
(51) Int. Cl.: G16B 20/00, G16B 30/00, G16B 40/00, C12Q 1/6827

(54) **METHOD FOR INTERROGATING MIXTURES OF NUCLEIC ACIDS**
VERFAHREN ZUR INTERROGATION VON NUKLEINSÄUREMISCHUNGEN
PROCÉDÉ POUR INTERROGER DES MÉLANGES D'ACIDES NUCLÉIQUES

(30) Priority: 30.06.2015 GB 201511445
(43) Date of publication of application: 09.05.2018
(73) Proprietor: The Secretary of State for Defence, Salisbury, Wiltshire SP4 0JQ (GB)
(72) Inventor: PAYNE, Phillippa Maria, Salisbury Wiltshire SP4 0JQ (GB); STROUD, Jake Patrick, Salisbury Wiltshire SP4 0JQ (GB)
(74) Representative: Farnsworth, Alastair Graham
(86) International application number: PCT/GB2016/000135
(87) International publication number: WO 2017/001813

(56) References cited:
- EP-A2- 1 229 135
- WO-A2-2012/177817
- US-A1- 2009 226 916
- US-A1- 2009 270 264
- US-A1- 2010 198 522
- US-A1- 2011 093 208
- US-A1- 2015 024 378
- TSEWEI WANG ET AL: "Least-Square Deconvolution: A Framework for Interpreting Short Tandem Repeat Mixtures", JOURNAL OF FORENSIC SCIENCES, vol. 51, no. 6, 1 November 2006 (2006-11-01), pages 1284-1297, XP055304351, CHICAGO, IL, US ISSN: 0022-1198, DOI: 10.1111/j.1556-4029.2006.00268.x

## Description

The present application is concerned with methods for interrogating mixtures of nucleic acids through amplification of short tandem repeat markers (loci) within each nucleic acid, and thereby analysis of the amounts of each allele amplified from each marker, and in particular interrogating mixtures of DNA, such as forensic (trace) samples, to identify the most probable number of contributors of nucleic acid in the mixture, the most probable ratio/proportion of the nucleic acids in the mixture, and thereby the most probable nucleic acid sequence for each marker within a nucleic acid.

Forensic DNA analysis was first developed in about 1985. The development of methods utilising short tandem repeat markers (loci) began in the early 1990s. An STR locus is a length polymorphism where alleles have different numbers of short DNA units (typically four or five base pairs) that are repeated in tandem. An allele is one of two or more versions of a gene. An individual inherits two alleles for each gene, one from each parent. If the two alleles are the same, the individual is homozygous for that gene. If the alleles are different, the individual is heterozygous. When a polymorphic locus has 15 or more possible alleles it provides for over a hundred possible genotype values, and thus is useful for distinguishing between people in a population.

The analysis of complex DNA mixtures, particularly those containing several DNA profiles remains a challenge, with statistical and mathematical models within software being used to improve the sensitivity and accuracy of analysis.

Generally, the presence of multiple contributors is identified through maximum allele count, based on each person having two alleles per marker (locus), and thus the identification of a maximum of four alleles for any one marker would be indicative of a mixture of DNA from two contributors. This is however based on a potentially dangerous assumption, as the minimum number of alleles for any mixture is one, since each contributor could potentially have two copies of the same allele. Thus, when ≤ 2 alleles are observed at any locus, a sample may still present a DNA mixture. Moreover, mixtures are still currently interpreted by an expert DNA analyst, as opposed to through using objective algorithmic methods.

Patent application US 2011/093208 discloses a computer implemented method to resolve DNA mixture for forensic application that involves calculating allele peak fit for possible genotype combination at former locus and subsequent locus.

Accurate statistical interpretation of mixtures of DNA remains a challenge, and there is especially a need in the art for methods which do not rely upon assumptions, such as the number of contributors from maximum allele count. A method that could identify the number of contributors, and DNA sequences for the STR markers therein, without any knowledge of the contributors, or the contributors' genotypes, would be of great benefit. Such a method could avoid biased analysis based on the known identification of one or more potential contributors, such as a victim or suspect.

Currently it is particularly challenging to resolve mixtures comprising nucleic acid from three contributors, especially in an unbiased analysis, and thus not reliant on whether potential nucleic acid sequences are known or not.

The present invention thus generally aims to provide an unbiased means for interrogating mixtures of nucleic acids in a sample, especially mixtures of DNA in a forensic sample, which can in particular interrogate mixtures of nucleic acid from three, or more, contributors.

Thus, in a first aspect, the present invention provides a method for interrogating a mixture of nucleic acids in a sample through analysis of short tandem repeat markers to identify the most probable proportion of each nucleic acid in the sample for a defined number of contributors, and the most probable allele sequences for each marker within each nucleic acid from each contributor comprising
I. providing a sample which may comprise a mixture of nucleic acids for interrogation;
II. amplifying multiple short tandem repeat markers from nucleic acids in the sample to enable amplification of a maximum of two alleles per marker per nucleic acid;
III. evaluating data from the amplification such that the number of alleles per marker in the sample, and amounts and relative percentages of each allele per marker in the sample are ascertained;
IV. identifying all possible allele pair combinations per marker in the sample from the data;
V. Predicting the amount and relative percentages of each allele for each possible allele pair combination for each marker in the sample for a defined number of contributors in various proportions;
VI. Comparing, and calculating the residual (i.e. difference) between the relative percentages of each allele per marker in the sample with that predicted for each possible allele pair combination for each marker for a defined number of contributors in various proportions, and using least square analysis to minimise the sum of squared residuals obtaining the probability for each allele combination for each marker for the defined number of contributors being present in the sample at each proportion, and calculating the mode and median from each residual to check for a consistent mixture proportion; wherein steps II to VI are repeated numerous times, wherein the numerous times is at least ten, and the probabilities from each repetition for each allele combination for each marker for the defined number of contributors at each proportion are multiplied to identify the most likely allele pair combinations and their most likely proportion in the sample for each marker, and thereby identifying the most likely proportion of nucleic acids in the sample for the defined number of contributors, and the most likely allele sequences for each marker within each nucleic acid from each contributor.

The Applicant has created a method for interrogating a mixture of nucleic acid in a sample through analysis of short tandem repeat (STR) markers (loci) to identify the most probable proportion of each nucleic acid in the sample based solely on the amount of each allele with no knowledge of the contributors, or the contributors' genotypes. This method does not rely upon assumptions, such as the number of contributors in a sample. This method does not require allelic frequency tables or population statistics. It does not require the number of contributors to the mixture sample to be known.

The method is undertaken a number of times for different defined number of contributors to identify the most likely proportion of nucleic acids in the sample and thereby the most likely number of contributors. For example the method may be undertaken with the defined number of contributors being, one, two, three, and four, to statistically identify the most likely number of contributors and the most likely proportion of nucleic acids in the sample.

The method relies upon minimising the residuals between the predicted/estimated amount and the observed amount for each allele value across all markers.

The method is designed to identify the, proportion of nucleic acid for each contributor in the mixture, and the most likely allele sequences for each marker for each contributor. The method allows for an unbiased analysis of nucleic acid mixtures, which is advantageous since genotypic information is often not available for the potential contributors.

Once a potential contributor's genotypes are known, we can compare them to those produced from the unbiased analysis of the mixture and produce a statement such as 'the evidence supports the contention that genotype combination AB, CD is the most likely'.

Background allele frequencies can also be incorporated to produce a Likelihood Ratio, by following the methods of Evett et al, 1991, Journal of the Forensic Science Society, Volume 31, Issue 1, pages 41-47, that someone contributed to the mixture.

Preferably the sample is a forensic sample, such as a trace forensic sample.

Differentiation is in particular directed to identifying the most probable number of contributors of nucleic acid in the sample (i.e. sources of nucleic acid), and the most probable allele sequences for each marker within each nucleic acid.

Although step II. is directed to amplification of two alleles per marker per nucleic acid because each contributor will have one allele from each parent, it may be that the two alleles are the same. If the alleles are the same for a particular marker, the individual is homozygous for that marker. If the alleles are different, the individual is heterozygous.

Multiple short tandem repeat (STR) markers are at least two, but most likely at least ten, such as between 10 and 16 STR markers.

Evaluating data may be enabled through the production of an electropherogram, such that the number of alleles per marker amplified in the sample, and the respective peak area and/or peak height of each allele, can be ascertained/calculated. The amounts of each allele are thus preferably represented by peak height and/or peak area for each alelle in an electropherogram, and the establishment of the relative amount of each allele per marker by dividing each peak height and/or peak area by the sum of the peak heights and/or peak areas of each allele per marker. An electropherogram is a plot of results from an analysis done by electrophoresis based sequencing. An advantage of the method is that it can utilise not only the peak height but the peak area of the allelic signature produced via an electropherogram.

The step of comparing the relative percentages of each allele per marker with that predicted for each possible allele pair combination for each marker for a defined number of contributors in various proportions, may involve comparing the percentages with that predicted for two, three, four or five contributors, thus the defined number of people may be two, three, four or five, or more.

This step of the method may also be repeated for different defined numbers of contributors.

Alternatively, the method could interrogate the sample based on a number of possible contributors, such as two or three contributors, to enable identification of the most likely number of contributors to a mixture of nucleic acid, together with the probable proportion of each nucleic acid in the sample, and the most probable allele nucleic acid sequences for each marker within each nucleic acid for each contributor. An advantage of the method is its ability to determine the number of contributors to a mixture. The analysis based on numerous defined numbers of contributors may require the method to be performed successively or sequentially with each defined number of contributors.

The term various proportions relates to the possible ratios of concentration of nucleic acids in the sample. For two contributors the various proportions may differ from between 99:1 (or 1:99) between the two contributors, to an equal proportion of 50:50, with proportions varying in increments of 1, or 5, or 10, for example the various proportions could range from 5:95 (or 95:5) to 50:50, in increments of 5. For three contributors, the various proportions may range from 1:1:98 (or 1:98:1, or 98:1:1) through to equal proportions from each contributor. The increments may again vary in increments of 1, or 5, or 10. For example, the ranges may vary from 5:5:90 to 30:30:35 in increments of 5.

The step of calculating the residual between the actual relative percentage of alleles per marker and that predicted for each allele pair combination for each marker for a defined number of contributors in the various proportions searches for a consistent mixture proportion across all markers, searching for a low residual for at least some combinations of allele pairs.

Step VI. of the method may be achieved by creating a Chi-square test statistic based on the residual difference between the predicted amount (or percentage) and the actual amount (or percentage) for each allele, considering each allele pair combination and mixture proportion such as described in Curran et al, 2008, Science and Justice, Volume 48, Issue 4, pages 168-177.

The analysis may comprise incorporating a normalised threshold for each marker, where any residuals within α of the minimum residual at that marker (locus) are used to determine a possible mixture proportion. The value for α may be 0.05 (thus 5% around the minimum difference), or 0.1 (thus 10% around the minimum difference), which could be displayed for example as a Gaussian distribution plot to enable identification of the most probable proportion of each allele combination per marker in the sample. The Applicant has observed that low residuals tend to cluster around the 'true' mixture proportion, and a Gaussian shaped distribution is observed over the 'true' mixture proportion.

Optionally, parameters such as mode, median and mean can be calculated to check for a consistent mixture proportion, and ensure minimal residuals, for each data set, and particularly use of combinations of parameters, such as calculating both mode and median.

The value of the data and probability will be more robust the more repetitions that can be undertaken. The numerous times are at least 10, or at least 100, though more likely at least 500 or at least 1000. The number of times undertaken may depend on the amount of data to be processed, and thus more times could be possible where the defined number of contributors is two, rather than three. For an analysis based on two potential contributors the numerous times may be 10,000, whereas for three potential contributors the numerous times may be 1,000.

For said step if the product of all probabilities is >0.5 for specific allele pair combinations at a particular proportion then that is most likely the correct proportion for that marker, and thereby the most likely proportion of nucleic acids in the sample.

The most likely allele sequences for each marker within each nucleic acid are consequently inferred from the most likely proportion of each allele combination for each marker. The mixture proportion with highest likelihood can be inferred when the residuals for all markers simultaneously minimise.

The method enables a user to search for a consistent mixture proportion across all markers with a low residual for at least some combination of allele pairs.

The advantage of using this approach to calculate the minimum residuals is that the analysis can support the original inference of the expert by considering all possible mixture combinations without any prior conditioning on a genotype combination or mixture proportion.

The present invention will now be described with reference to the following non-limiting examples and drawings in which
Figure 1 illustrates contour plots of the residuals produced from the Curran et al data for the first 6 loci (a) and the last seven loci (b);
Figure 2 displays the user prompt in the tool to adjust the threshold parameter, and also a graphical representation of the multinomial distribution produced, with peaks above 0.3 and 0.7;
Figure 3 is a graphical representation of the Gaussian distributions produced from the Curran data, where a standard deviation of 0.05 was used;
Figure 4 is a graphical representation of the probabilities attributed to the most likely genotypes that created the mixture from the Curran et al data;
Figure 5 displays the user prompt in the tool to adjust the threshold parameter, and also a graphical representation of the multivariate normal distribution produced, with peaks above 0.3 and 0.7;
Figure 6 is a graphical representation of the Gaussian distributions produced for the Perlin et al data, with a standard deviation of 0.05;
Figure 7 is a graphical representation of the probabilities attributed to the most likely genotypes that created the mixture from the Perlin et al data;
Figure 8 is a graphical representation of the pre-amplification mixture proportion estimation for Example 3 if two people were to be represented in the mixture;
Figure 9 is a graphical representation of the pre-amplification mixture proportion estimation for Example 3 if three people were to be represented in the mixture; and
Figure 10 is a graphical representation of the probabilities attributed to the most likely genotypes that created the mixture from the data in Example 3.

### Examples

### Two Person Mixtures

### Example 1

The Applicant illustrates the method using possible allele pair combinations taken from Curran et al (2008, Science and Justice, Volume 48, Issue 4, pages 168-177) at locus (marker) D3S1359.

**Table 1. Data from Curran et al pertaining to a 2 person mixture.**

| Locus | Alleles in the mixture | Allele Peak Area | True Genotype Combination | |
|---|---|---|---|---|
| | | | Victim | Offender |
| D3S1358 | 15 | 1989 | 15 | 15 |
| | 16 | 739 | 16 | |
| | 18 | 1550 | | 18 |
| vWA | 15 | 1318 | | 15 |
| | 16 | 621 | 16 | |
| | 18 | 793 | 18 | |
| | 19 | 1200 | | 19 |
| FGA | 21 | 2414 | 21 | 21 |
| | 22 | 1461 | | 22 |
| | 23 | 687 | 23 | |
| D8S1179 | 12 | 1431 | | 12 |
| | 13 | 603 | 132 | |
| | 14 | 560 | 14 | |
| | 16 | 986 | | 16 |
| D21S11 | 28 | 1410 | | 28 |
| | 30 | 1199 | 30 | |
| | 32.2 | 1506 | | 32.2 |
| D18S51 | 12 | 471 | 12 | |
| | 13 | 386 | 13 | |
| | 17 | 1181 | | 17 |
| | 18 | 1029 | | 18 |
| D5s818 | 12 | 2561 | 12 | 12 |
| | 13 | 463 | 13 | |
| D13S317 | 11 | 1607 | 11 | 11 |
| | 12 | 834 | | 12 |
| D7S820 | 8 | 723 | | 8 |
| | 10 | 1203 | 10 | 10 |
| | 11 | 289 | 11 | |
| D16S539 | 11 | 1262 | | 11 |
| | 12 | 515 | 12 | |
| | 13 | 1253 | | 13 |
| | 14 | 514 | 14 | |
| THO1 | 5 | 944 | | 5 |
| | 6 | 935 | | 6 |
| | 8 | 633 | 8 | |
| TPOX | 8 | 1257 | 8 | 8 |
| | 10 | 984 | | 10 |
| | 11 | 447 | 11 | |
| CSF1PO | 10 | 482 | 10 | |
| | 11 | 697 | | 11 |
| | 12 | 617 | | 12 |

At this locus, the observed alleles were 15, 16 and 18. This gives 6 possible (unordered) pairs of allele values: 15/15, 15/16, 15/18, 16/16, 16/18 and 18/18. Subsequently, this produces 12 possible ordered combinations of these pairs for 2 people, (since the total combination of allele values must be identical to those observed in the mixture which would exclude, for example, 15/15 for contributor 1 and 15/16 for contributor 2, since allele 18 is neglected here). The ordered pairs are shown in Table 2.

**Table 2. Possible allele pair combinations derived from the data for locus D3S1359 as shown in Table 1.**

| Contributor 1 | Contributor 2 |
|---|---|
| 15 15 | 16 18 |
| 16 18 | 15 15 |
| 15 16 | 15 18 |
| 15 18 | 15 16 |
| 15 16 | 16 18 |
| 16 18 | 15 16 |
| 15 16 | 18 18 |
| 18 18 | 15 16 |
| 15 18 | 16 16 |
| 16 16 | 15 18 |
| 15 18 | 16 18 |
| 16 18 | 15 18 |

We then calculate all possible (non-symmetric) mixture proportions in increments of 0:05. In this example, for a 2 person mixture this was 10 possible mixture proportions from 0.05:0.95 to 0.5 and 0.5.

It should be noted that, possibly counter-intuitively, greater resolution achieved by using smaller increments than 0.05, did not increase the sensitivity of the model. This is due to the inherent variation displayed in mixtures which in part is a result of the PCR process.

We can then calculate the expected peak area for each allele value, mixture proportion and combination of allele pairs across all loci. As done by Curran et al (2008, Science and Justice, Volume 48, Issue 4, pages 168-177), we can create a Chi-square test statistic for each allele pair combination and mixture proportion.

The list of possible combinations of allele pairs is used at this stage as a parameter to expose a consistent mixture proportion. The developed methodology searches for a consistent mixture proportion across all loci with a low residual for some combination of allele pairs. The mixture proportion with highest likelihood can be inferred when the residuals of all loci simultaneously minimise. The advantage of using this approach to calculate the minimum residuals is that the analysis can support the original inference of the expert by considering all of the possible mixture combinations without any prior conditioning on a genotype combination or mixture proportion.

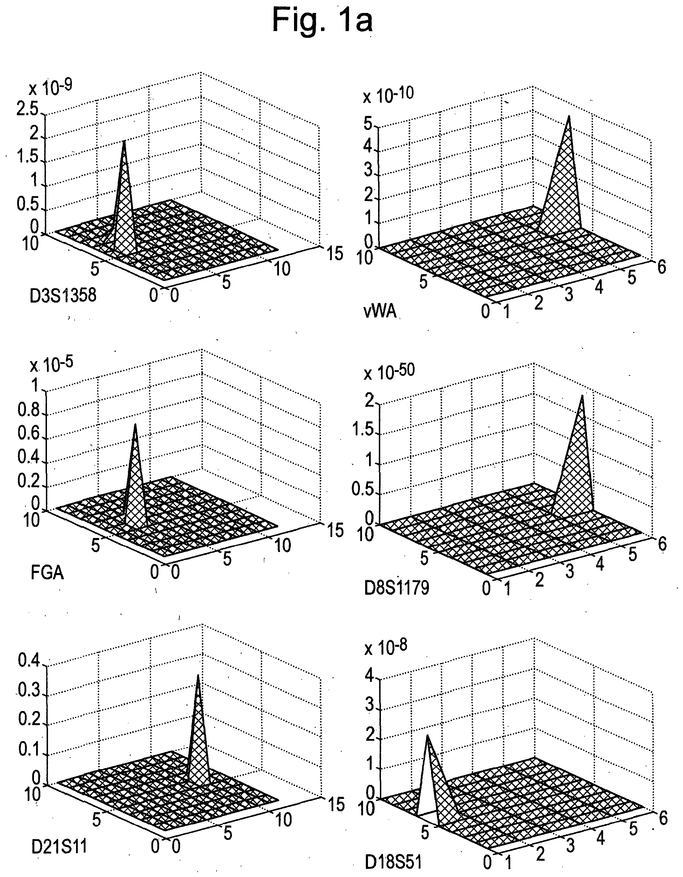

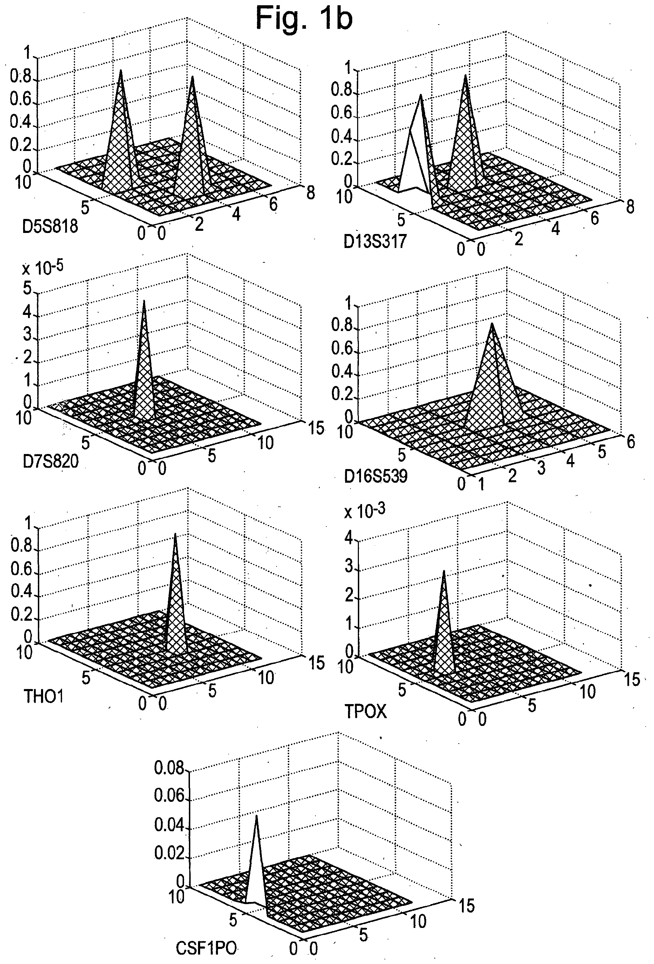

Having regard to Figure 1, the data can be represented as a visual representation of the matrix for each locus, where the Chi-square statistic has been inverted into a Chi-square distribution to produce peaks rather than troughs for display purposes.

From these surface plots we can see that the 6th mixture proportion, which in this case corresponds to a ratio of 3:7, produces a consistently low residual across all loci.

The developed methodology can identify a consistent mixture proportion by using a normalised threshold method at each locus where any residuals within α of the minimum residual at that locus are used to determine a possible mixture proportion. The value for α in this example is 0.1 although this parameter can be adjusted. In fact from the results of using this method, certainly for simple (2 person) mixtures, other low residuals at a locus appear to cluster around the 'true' mixture proportion, indicating that a threshold method is desirable in determining the mixture proportion.

The mode and median are then calculated and some sensitivity testing is employed to check for a consistent mixture proportion.

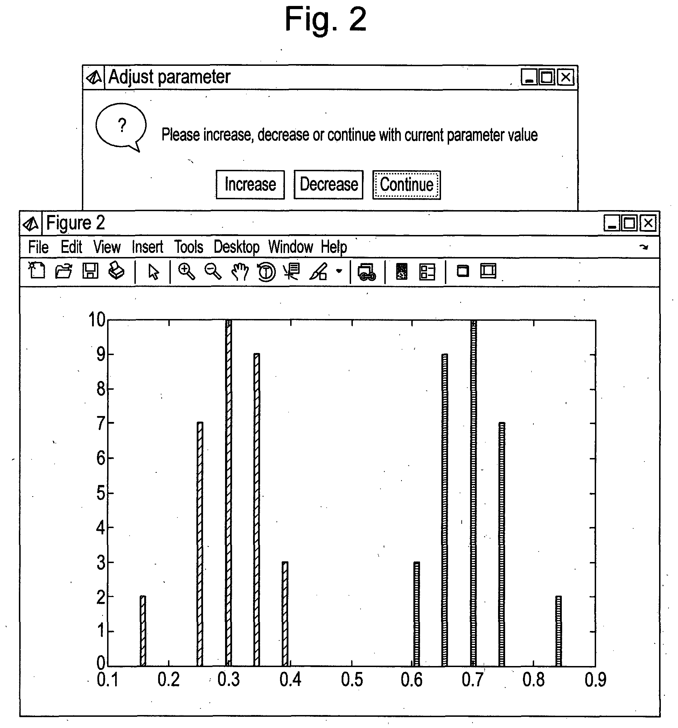

Having regard to Figure 2, the results can be represented as a histogram of mixture proportions for residuals within α (0.1) of the minimum residual at each locus. Clearly the minimum number of mixture proportions that can be identified would be, in this case, 13 since there are 13 loci. We have noted that in some cases, the minimum residual at a locus will not correspond to the 'correct' mixture proportion, however we have also observed that low residuals tend to cluster around the 'true' mixture proportion and a Gaussian shaped distribution is observed over the 'true' mixture proportion. It is thus recommended to set α to between 0 and 0:1. It should be noted that a value of 0.1 for α has identified the correct (known) mixture proportion in all analyses performed.

Having regard to Figure 2, a Gaussian shaped distribution, although symmetric since mixture proportions must sum to 1, is produced with peaks over 0.3 and 0.7 which is indicative of a mixture proportion of 30% for the minor contributor and 70% for the major contributor. This part of the analysis can also clearly provide insight into the number of contributors to the mixture - i.e. for a predefined number of contributors of two, is there a clear Gaussian distribution about two values within the plot, and do the values sum to 1 (i.e. 100%).

Once the mixture proportion had been estimated, the next step was to analyse the most likely genotypes that produced the mixture for the specific estimated mixture proportion (i.e. 30:70). Our method utilises sampling of mixture proportions from a Gaussian distribution with a mean provided by the estimated mixture proportion and standard deviation of 0:05, to account for the variability observed in mixture proportions across loci.

After each analysis, the combination of genotypes producing the minimum residual were selected. This was performed simultaneously across all loci providing a probability that a genotype combination contributed to the mixture (if enough analyses are used) for each locus. We set simulations to 10,000 for two person mixtures and 1,000 for three person mixtures for time considerations.

The algorithm produced to undertake the calculations takes several seconds for a two person mixture and under a minute for a three person mixture.

Genotype combinations are then ranked from most likely to least likely and a joint probability likelihood can also be constructed if necessary to provide a likelihood across all loci.

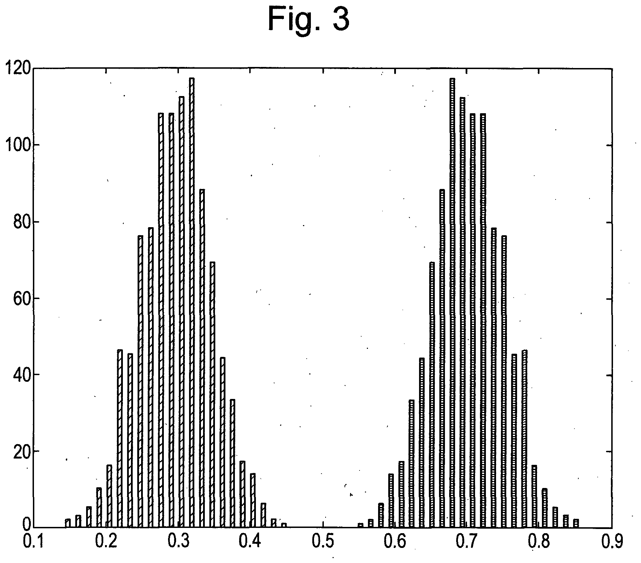

Having regard to Figure 3, the Gaussian sampling distributions generated for this specific data is shown, with the standard deviation of 0.05 used. The number of times a genotype combination is identified as having the minimum residual can be interpreted as a probability if divided by the total number of simulations used.

For this data the analysis correctly identified all genotypes as being the highest ranked genotypes with a mixture proportion of 3:7.

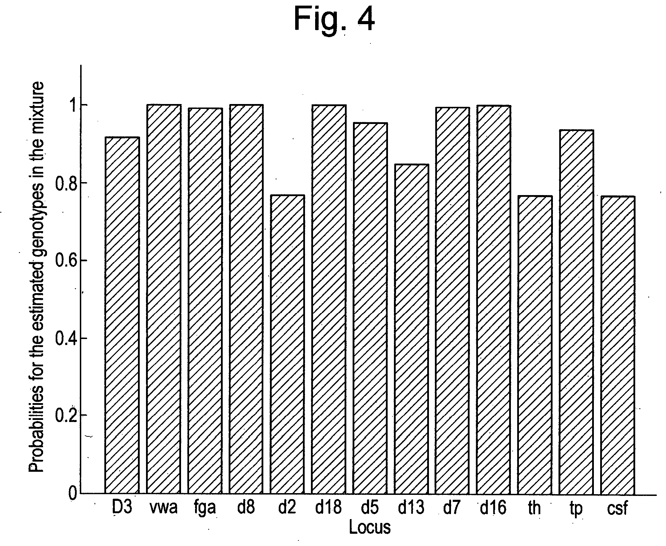

Having regard to Figure 4, the probabilities that the identified genotypes are the true genotypes of the two profiles that produced the mixture are shown, and are also detailed in Table 3.

**Table 3. The probabilities attributed to the most likely genotypes that created the mixture from the data for the mixture proportion. The genotypes identified correspond to the known victim and offender genotypes at every locus.**

| locus | Genotype for minor contributor | Genotype for major contributor | Probability |
|---|---|---|---|
| 'D3' | 15 16 | 15, 18 | 0.918 |
| 'vwa' | 16, 18 | 15, 19 | 1 |
| 'fga' | 21, 23 | 21, 22 | 0.99 |
| 'd8' | 13, 14 | 12, 16 | 1 |
| 'd2' | 30, 30 | 28, 32.2 | 0.769 |
| 'd18' | 12, 13 | 17, 18 | 1 |
| 'd5' | 12, 13 | 12, 12 | 0.956 |
| 'd13' | 11, 11 | 11, 12 | 0.849 |
| 'd7' | 10, 11 | 8, 10 | 0.996 |
| 'd16' | 12, 14 | 11, 13 | 1 |
| 'th' | 8, 8 | 5, 6 | 0.769 |
| 'tp' | 8, 11 | 8, 10 | 0.94 |
| 'csf' | 10, 10 | 11, 12 | 0.769 |

### Example 2

The method was performed on data obtained from Perlin et al, 2011, Journal of Forensic Sciences, Volume 56, Issue 6, pages 1430-1447., which article was concerned with a validation of TrueAllele.

**Table 4. Data from Perlin et al obtained by STR amplification of particular markers (loci), as derived from peak area of electropherograms.**

| Locus | Allele Value | Peak Area |
|---|---|---|
| d2 | 16 | 1339 |
| d2 | 18 | 2992 |
| d2 | 20 | 1947 |
| d2 | -1 | 3722 |
| d3 | 14 | 5010 |
| d3 | 15 | 4990 |
| d8 | 9 | 2832 |
| d8 | 12 | 1426 |
| d8 | 13 | 3829 |
| d8 | 14 | 1913 |
| d16 | 11 | 6801 |
| d16 | 13 | 1607 |
| d16 | 14 | 1593 |
| d18 | 12 | 1504 |
| d18 | 13 | 3290 |
| d18 | 14 | 3443 |
| d18 | 17 | 1764 |
| d19 | 12.2 | 3109 |
| d19 | 14 | 3092 |
| d19 | 15 | 3799 |
| d21 | 27 | 1289 |
| d21 | 29 | 3913 |
| d21 | 30 | 4798 |
| fga | 19 | 4621 |
| fga | 24 | 1561 |
| fga | 25.2 | 3817 |
| th | 6 | 1268 |
| th | 7 | 4691 |
| th | 9 | 4041 |
| vwa | 17 | 7265 |
| vwa | 18 | 2735 |

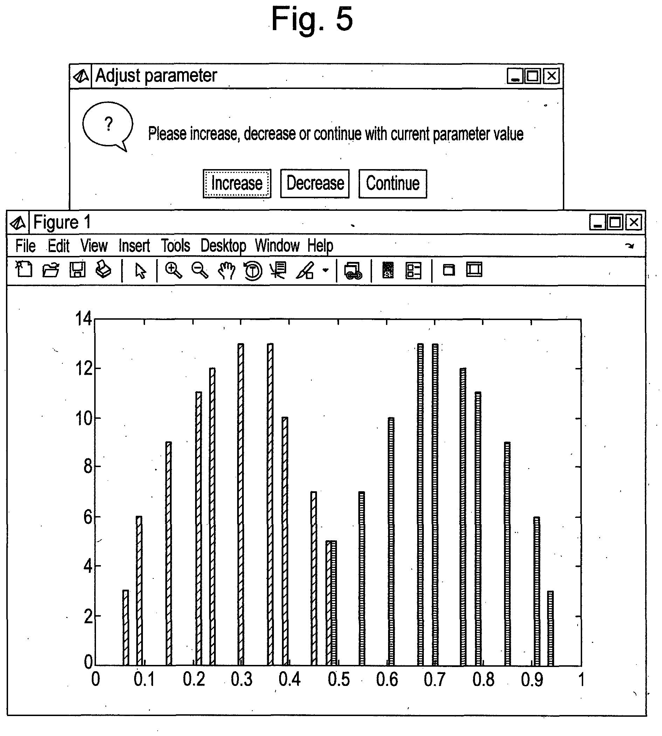

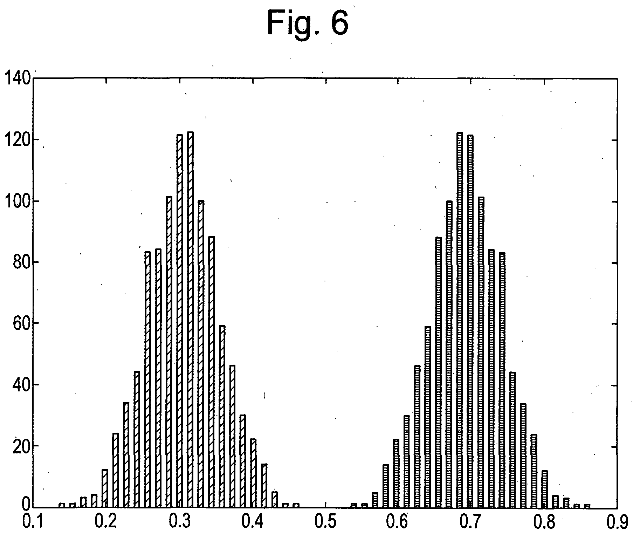

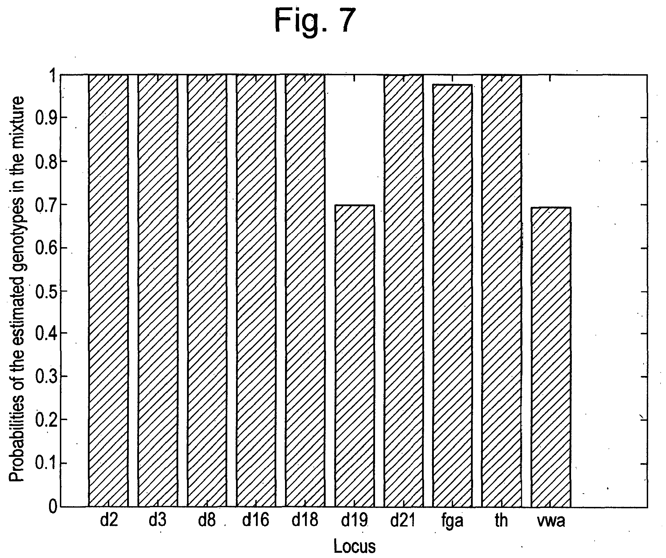

Having regard to Figure 5 and Figure 6, the estimated mixture proportion, and the Gaussian distribution for the data as evaluated by the method is displayed. Having regard to Figure 7, the probabilities of the most likely genotypes across all loci are displayed with the correct genotypes being identified at all loci. The genotypic information is displayed in Table 5 along with the probability, and joint probability. This can be compared to the results produced by Cowell et.al, 2007, Forensic Science International, Volume 166, Issue 1, pages 28-34, where all the correct genotypes were identified for one (of 4 provided) parameter and model configurations. The joint probability for our model is also higher than that produced by Cowell et.al (0.256704).

**Table 5. The result of applying the method on the Perlin et al data.**

| Locus | Allele Pair Contributor 1 | Allele Pair Contributor 2 | Probability |
|---|---|---|---|
| d2 | 16, 20 | 18, 21 | 1 |
| d3 | 14, 15 | 14, 15 | 1 |
| d8 | 12, 14 | 9, 13 | 1 |
| d16 | 13, 14 | 11, 11 | 1 |
| d18 | 12, 17 | 13, 14 | 1 |
| d19 | 14, 14 | 12.2, 15 | 0.697 |
| d21 | 27, 30 | 29, 30 | 0.996 |
| fga | 19, 24 | 19, 25.2 | 0.977 |
| th | 6, 7 | 7, 9 | 0.996 |
| vwa | 18, 18 | 17, 17 | 0.694 |
| Joint | | | 0.4688 |

### Three Person Mixtures

### Example 3 - Simulated Three Person Mixture

We simulated data across 10 markers for a three person mixture. We used a mixture proportion of [0.2, 0.3, 0.5] as a random choice. We present the data set in Table 6.

**Table 6. Data for the simulated three person mixture.**

| Locus | Allele Value | Peak Area |
|---|---|---|
| d3 | 7 | 450 |
| d3 | 8 | 1300 |
| d3 | 9 | 1750 |
| d3 | 10 | 1250 |
| d5 | 10 | 1450 |
| d5 | 11 | 355 |
| d5 | 5 | 2222 |
| d7 | 3 | 290 |
| d7 | 4 | 300 |
| d7 | 5 | 455 |
| d7 | 6 | 1222 |
| d7 | 7 | 754 |
| d8 | 4 | 2200 |
| d8 | 5 | 2600 |
| d8 | 6 | 1100 |
| d8 | 7 | 2000 |
| d13 | 4 | 100 |
| d13 | 5 | 500 |
| d13 | 6 | 300 |
| d18 | 1 | 500 |
| d18 | 2 | 3000 |
| d18 | 5 | 1800 |
| d21 | 3 | 1900 |
| d21 | 4 | 500 |
| d21 | 7 | 500 |
| fga | 1 | 510 |
| fga | 2 | 720 |
| fga | 3 | 450 |
| fga | 4 | 320 |
| vwa | 5 | 1000 |
| vwa | 6 | 600 |
| vwa | 7 | 3000 |
| vwa | 9 | 1700 |
| vwa | 8 | 550 |
| tho | 1 | 1250 |
| tho | 2 | 700 |
| tho | 3 | 1200 |
| tho | 4 | 600 |

We applied both the normal and light version of the tool to this data set. The light version of the tool does not allow for adjustment of the parameter α to determine the pre-amplification ratio and performs only one simulation to estimate the most likely genotypes that created the mixture. The light version of the tool does not fit a distribution to the estimated pre-amplification mixture proportion but merely ranks the residuals for the estimated pre-amplification mixture proportion. Therefore we cannot attribute probabilities to the final output but produce a list say of the 5 most likely genotypes that produced the mixture at each locus. We also applied the normal version of the tool.

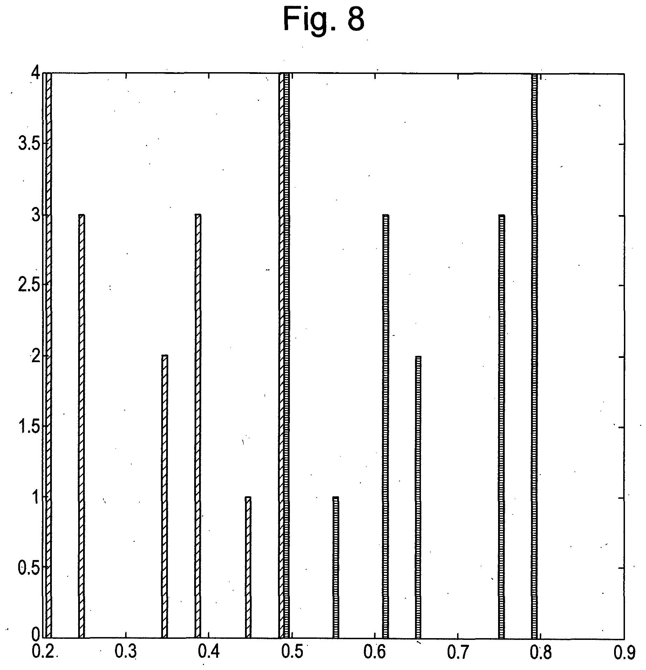

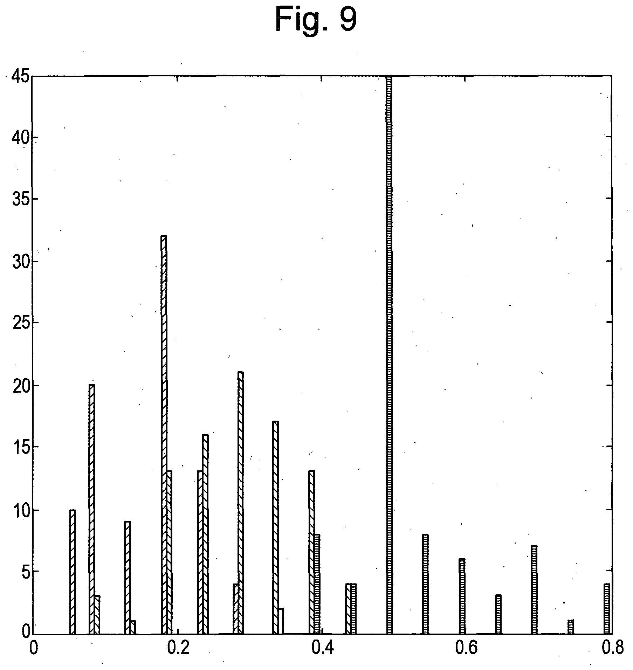

Having regard to Figures 8 and 9, the distributions found for the pre-amplification mixture proportion when two (Figure 8) and three contributors (Figure 9) are considered is shown. For two contributors scenario it can be seen that there are no symmetric distributions, and no strong distribution. We can however see from Figure 9 that Gaussian distributions occur over 0.2, 0.3, and 0.5, and thus that a three person mixture is most likely, with mixture proportion 0.2, 0.3 and 0.5. We present the most likely genotypes expected to produce this mixture in Table 7. We have listed them from the most likely to the 4th most likely. We use bold italics to indicate classification errors. We can see that by the 4th combination we have no classification errors. In fact, the most likely genotype combination correctly identifies 7 of the 10 markers first time.

**Table 7. Genotypic combination results for the three person mixture, descending from most likely to fourth most likely. Bold italics are used to indicate incorrect identifications.**

| Most likely | First Person | Second Person | Third Person |
|---|---|---|---|
| d3 | ***8,10*** | ***9, 10*** | ***8, 9*** |
| d5 | 10, 11 | 5, 5 | 10, 5 |
| d7 | 3, 4 | 6, 6 | 6, 7 |
| d8 | ***6, 6*** | ***4, 4*** | ***5, 5*** |
| d13 | 4, 5 | 5, 6 | 5, 6 |
| d18 | 5, 1 | 2, 2 | 5, 2 |
| d21 | 4, 4 | 3, 7 | 3, 3 |
| fga | 3, 2 | 3, 4 | 2, 1 |
| vwa | 6, 6 | 7, 7 | 7, 9 |
| tho | ***3, 1*** | ***3, 1*** | ***2, 1*** |

| Second most likely | | | |
|---|---|---|---|
| d3 | 8, 8 | 8, 9 | 9, 10 |
| d5 | 10, 11 | 5, 5 | 10, 5 |
| d7 | 3, 4 | 6, 6 | 6, 7 |
| d8 | ***6, 6*** | ***4, 4*** | ***5, 5*** |
| d13 | 4, 5 | 5, 6 | 5, 6 |
| d18 | 5, 1 | 2, 2 | 5, 2 |
| d21 | 4, 4 | 3, 7 | 3, 3 |
| fga | 3, 2 | 3, 4 | 2, 1 |
| vwa | 6, 6 | 7, 7 | 7, 9 |
| tho | ***3, 1*** | ***3, 1*** | ***2, 1*** |

| Third Most likely | | | |
|---|---|---|---|
| d3 | 8,8 | 8, 9 | 9, 10 |
| d5 | 10, 11 | 5, 5 | 10, 5 |
| d7 | 3, 4 | 6, 6 | 6, 7 |
| d8 | ***6, 6*** | ***4, 4*** | ***5, 5*** |
| d13 | 4, 5 | 5, 6 | 5, 6 |
| d18 | 5, 1 | 2, 2 | 5, 2 |
| d21 | 4, 4 | 3, 7 | 3, 3 |
| fga | 3, 2 | 3, 4 | 2, 1 |
| vwa | 6, 6 | 7, 7 | 7, 9 |
| tho | 3, 2 | 3, 2 | 1, 1 |

| Fourth Most Likely | | | |
|---|---|---|---|
| d3 | 8,8 | 8, 9 | 9, 10 |
| d5 | 10, 11 | 5, 5 | 10, 5 |
| d7 | 3, 4 | 6, 6 | 6, 7 |
| d8 | 5, 4 | 4, 6 | 5, 5 |
| d13 | 4, 5 | 5, 6 | 5, 6 |
| d18 | 5, 1 | 2, 2 | 5, 2 |
| d21 | 4, 4 | 3, 7 | 3, 3 |
| fga | 3, 2 | 3, 4 | 2, 1 |
| vwa | 6, 6 | 7, 7 | 7, 9 |
| tho | 3, 2 | 3, 2 | 1, 1 |

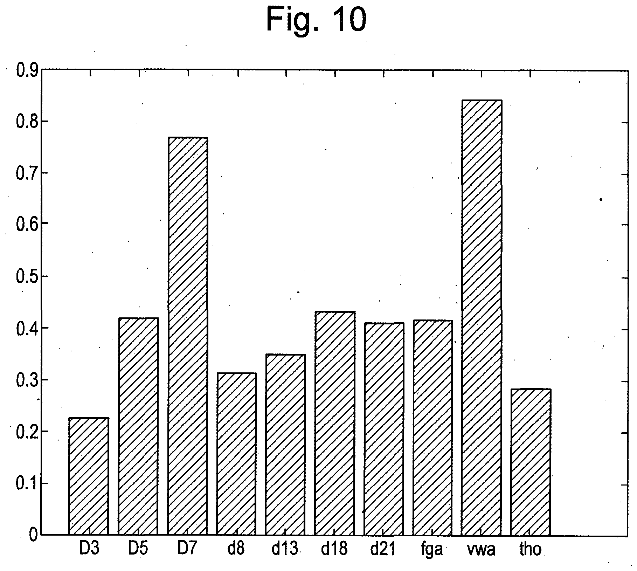

Having regard to Figure 10, the probabilities of the most likely genotype combinations are shown, as a result of running the normal version of the tool. The lowest probabilities here correspond to the mis-identified genotypes for the highest ranked contributor genotypes in Table 7. This is encouraging as the probabilities output from the normal version of the tool clearly provide a strong indication to the user that genotypes may have been mis-identified.

## Claims

1. A method for interrogating a mixture of nucleic acids in a sample through analysis of short tandem repeat markers to identify the most probable proportion of each nucleic acid in the sample for a defined number of contributors, and the most probable allele sequences for each marker within each nucleic acid from each contributor comprising
I. providing a sample which may comprise a mixture of nucleic acids for interrogation;
II. amplifying multiple short tandem repeat markers from nucleic acids in the sample to enable amplification of a maximum of two alleles per marker per nucleic acid;
III. evaluating data from the amplification such that the number of alleles per marker in the sample, and amounts and relative percentages of each allele per marker in the sample are ascertained;
IV. identifying all possible allele pair combinations per marker in the sample from the data;
V. predicting the amount and relative percentages of each allele for each possible allele pair combination for each marker in the sample for a defined number of contributors in various proportions;
VI. comparing, and calculating the residual (i.e. difference) between the relative percentages of each allele per marker in the sample with that predicted for each possible allele pair combination for each marker for a defined number of contributors in various proportions, and using least square analysis to minimise the sum of squared residuals obtaining the probability for each allele combination for each marker for the defined number of contributors being present in the sample at each proportion, and calculating the mode and median from each residual to check for a consistent mixture proportion ;
wherein steps II to VI are repeated numerous times, wherein the numerous times is at least ten, and the probabilities from each repetition for each allele combination for each marker for the defined number of contributors at each proportion are multiplied to identify the most likely allele pair combinations and their most likely proportion in the sample for each marker, and thereby identifying the most likely proportion of nucleic acids in the sample for the defined number of contributors, and the most likely allele sequences for each marker within each nucleic acid from each contributor.

2. A method according to Claim 1, wherein evaluating data is enabled through the production of an electropherogram, such that the number of alleles per marker amplified in the sample, and the respective peak area and/or peak height of each allele, can be ascertained and/or calculated, and the amounts of each allele are represented by the peak height and/or the peak area for each alelle in the electropherogram.

3. A method according to Claim 1 or Claim 2, wherein the defined number of contributors is two and the various proportions ranges from 95:5 to 50:50 in increments of 5.

4. A method according to Claim 1 or Claim 2, wherein the defined number of contributors is three and the various proportion ranges from 5:5:90 to 30:30:35 in increments of 5.

5. A method according to Claims 1 to 4, wherein Step Vi of the method is achieved by creating a Chi-square test statistic based on the residual difference between the predicted percentage and the actual percentage for each allele, considering each allele pair combination and each proportion.

6. A method according to Claims 1 to 5, wherein the residual in step vi includes data within α of the minimum residual for that marker, wherein α is 0.05, thus 5% around the minimum, or 0.1, thus 10% around the minimum.

7. A method according to Claims 1 to 6, wherein the numerous times is at least 100.

8. A method according to Claims 1 to 7, wherein the numerous times is at least 1000.

9. A method according to Claims 1 to 8, wherein the multiple short tandem repeat markers is at least 10 markers.

10. A method for interrogating a mixture of nucleic acids in a sample, wherein the method according to Claim 1 is performed successively or sequentially for numerous defined number of contributors to identify the most likely proportion of nucleic acids in the sample and thereby the most likely number of contributors, through the identification of the most likely allele pair combinations and their most likely proportion in the sample for each marker, and thereby the most likely allele sequences for each marker within each nucleic acid from each contributor.

## Patentansprüche

1. Verfahren zum Abfragen einer Mischung von Nukleinsäuren in einer Probe durch Analyse kurzer Tandem-Wiederholungsmarker, um den wahrscheinlichsten Anteil jeder Nukleinsäure in der Probe für eine definierte Anzahl von Beitragenden und die wahrscheinlichsten Allelsequenzen für jeden Marker in jeder Nukleinsäure von jedem Beitragenden zu identifizieren, mit den Schritten
I. Bereitstellen einer Probe, die eine Mischung von Nukleinsäuren umfassen kann, zur Abfrage;
II. Amplifizieren mehrerer kurzer Tandem-Wiederholungsmarker aus Nukleinsäuren in der Probe, um die Amplifikation von maximal zwei Allelen pro Marker und Nukleinsäure zu ermöglichen;
III. Auswerten von Daten aus der Amplifikation, so dass die Anzahl der Allele pro Marker in der Probe sowie die Mengen und relativen Prozentsätze jedes Allels pro Marker in der Probe bestimmt werden;
IV. Identifizieren aller möglichen Allelpaarkombinationen pro Marker in der Probe aus den Daten;
V. Vorhersagen der Menge und der relativen Prozentsätze jedes Allels für jede mögliche Allelpaarkombination für jeden Marker in der Probe für eine definierte Anzahl von zu verschiedenen Anteilen Beitragenden;
VI. Vergleichen und Berechnen des Residuums (d.h. der Differenz) zwischen den relativen Prozentsätzen jedes Allels pro Marker in der Probe und dem für jede mögliche Allelpaarkombination für jeden Marker für eine definierte Anzahl von zu verschiedenen Anteilen Beitragenden vorhergesagten Wert, und Verwenden der Kleinste-Quadrate-Analyse, um die Summe der quadrierten Residuen zu minimieren und dabei für jede Allel-Kombination für jeden Marker und für die definierte Anzahl von Beitragenden die Wahrscheinlichkeit, in der Probe in jedem Anteil vorhanden zu sein, zu erhalten, und Berechnen des Modus und Medians von jedem Residuum, um auf ein konsistentes Mischungsverhältnis zu überprüfen;
wobei die Schritte II bis VI zahlreiche Male wiederholt werden, wobei die zahlreichen Male mindestens zehn sind, und die Wahrscheinlichkeiten aus jeder Wiederholung für jede Allelkombination für jeden Marker für die definierte Anzahl von Mitwirkenden in jedem Verhältnis multipliziert werden, um die wahrscheinlichsten Allelpaarkombinationen und ihren wahrscheinlichsten Anteil in der Probe für jeden Marker zu identifizieren und dadurch den wahrscheinlichsten Anteil an Nukleinsäuren in der Probe für die definierte Anzahl von Beitragenden und die wahrscheinlichsten Allelsequenzen für jeden Marker in jeder Nukleinsäure von jedem Beitragenden zu identifizieren.

2. Verfahren nach Anspruch 1, wobei das Auswerten von Daten durch die Erzeugung eines Elektropherogramms ermöglicht wird, so dass die Anzahl der in der Probe amplifizierten Allele pro Marker und die jeweilige Peakfläche und/oder Peakhöhe jedes Allels bestimmt und/oder berechnet werden kann, und die Mengen jedes Allels durch die Peakhöhe und/oder die Peakfläche für jedes Allel im Elektropherogramm dargestellt werden.

3. Verfahren nach Anspruch 1 oder Anspruch 2, bei dem die definierte Anzahl von Beitragenden zwei ist und die verschiedenen Proportionen in Schritten von 5 von 95:5 bis 50:50 reichen.

4. Verfahren nach Anspruch 1 oder Anspruch 2, bei dem die definierte Anzahl von Beitragenden drei beträgt und die verschiedenen Proportionen in Schritten von 5 von 5:5:90 bis 30:30:35 reichen.

5. Verfahren nach den Ansprüchen 1 bis 4, wobei Schritt VI des Verfahrens durch Erstellen einer Chi-Quadrat-Teststatistik basierend auf der Restdifferenz zwischen dem vorhergesagten Prozentsatz und dem tatsächlichen Prozentsatz für jedes Allel unter Berücksichtigung jeder Allelpaar-Kombination und jeder Proportion erreicht wird.

6. Verfahren nach den Ansprüchen 1 bis 5, bei dem der Rest in Schritt vi Daten in einem Abstand α vom minimalen Residuum für diesen Marker beinhaltet, wobei α 0,05, also 5% um das Minimum, oder 0,1, also 10% um das Minimum, ist.

7. Verfahren nach den Ansprüchen 1 bis 6, bei dem die zahlreichen Male mindestens 100 betragen.

8. Verfahren nach den Ansprüchen 1 bis 7, bei dem die zahlreichen Male mindestens 1000 betragen.

9. Verfahren nach den Ansprüchen 1 bis 8, bei dem die mehreren kurzen Tandem-Wiederholungsmarker mindestens 10 Marker sind.

10. Verfahren zum Abfragen einer Mischung von Nukleinsäuren in einer Probe, wobei das Verfahren nach Anspruch 1 nacheinander oder sequentiell für eine Vielzahl von definierten Beitragenden durchgeführt wird, um den wahrscheinlichsten Anteil von Nukleinsäuren in der Probe und damit die wahrscheinlichste Anzahl von Beitragenden zu identifizieren, indem die wahrscheinlichsten Allelpaarkombinationen und ihr wahrscheinlichster Anteil in der Probe für jeden Marker und damit die wahrscheinlichsten Allelsequenzen für jeden Marker innerhalb jeder Nukleinsäure von jedem Beitragenden identifiziert werden.

## Revendications

1. Procédé pour interroger un mélange d'acides nucléiques dans un échantillon par analyse de marqueurs courts répétés en tandem pour identifier la proportion la plus probable de chaque acide nucléique dans l'échantillon pour un nombre défini de contributeurs, et les séquences alléliques les plus probables pour chaque marqueur dans chaque acide nucléique provenant de chaque contributeur, comprenant
I. l'obtention d'un échantillon qui peut comprendre un mélange d'acides nucléiques pour une interrogation ;
II. l'amplification de multiples marqueurs courts répétés en tandem provenant d'acides nucléiques dans l'échantillon pour permettre l'amplification au maximum de deux allèles par marqueur par acide nucléique ;
III. l'évaluation de données provenant de l'amplification telles que le nombre d'allyles par marqueur dans l'échantillon, et les quantités et pourcentages relatifs de chaque allèle par marqueur dans l'échantillon sont déterminés ;
IV. l'identification de toutes les combinaisons de paires d'allèles possibles par marqueur dans l'échantillon à partir des données ;
V. la prédiction de la quantité et des pourcentages relatifs de chaque allèle pour chaque combinaison de paire d'allèles possible pour chaque marqueur dans l'échantillon pour un nombre défini de contributeurs en diverses proportions ;
VI. la comparaison et le calcul de la valeur résiduelle (c'est-à-dire la différence) entre les pourcentages relatifs de chaque allèle par marqueur dans l'échantillon avec celle prédite pour chaque combinaison de paire d'allèles possible pour chaque marqueur pour un nombre défini de contributeurs en diverses proportions, et l'utilisation d'une analyse des moindres carrés pour minimiser la somme des valeurs résiduelles au carré, ce qui donne la probabilité pour chaque combinaison d'allèles pour chaque marqueur pour le nombre défini de contributeurs présents dans l'échantillon en chaque proportion, et le calcul du mode et de la médiane à partir de chaque valeur résiduelle pour vérifier une proportion de mélange cohérente ;
dans lequel les étapes II à VI sont répétées de multiples fois, dans lequel les multiples fois sont au moins dix, et les probabilités provenant de chaque répétition pour chaque combinaison d'allèles pour chaque marqueur pour le nombre défini de contributeurs en chaque proportion sont multipliées pour que soient identifiées les combinaisons de paires d'allèles les plus vraisemblable et leur proportion la plus vraisemblable dans l'échantillon pour chaque marqueur, moyennant quoi sont identifiées la proportion la plus vraisemblable d'acides nucléiques dans l'échantillon pour le nombre défini de contributeurs, et les séquences alléliques les plus vraisemblables pour chaque marqueur dans chaque acide nucléique provenant de chaque contributeur.

2. Procédé selon la revendication 1, dans lequel l'évaluation des données est permise par la production d'un électrophérogramme, si bien que le nombre d'allèles par marqueur amplifié dans l'échantillon, et la surface de pic et/ou la hauteur de pic respectives de chaque allèle, peuvent être déterminés et/ou calculés, et les quantités de chaque allèle sont représentées par la hauteur de pic et/ou la surface de pic pour chaque allèle dans l'électrophérogramme.

3. Procédé selon la revendication 1 ou la revendication 2, dans lequel le nombre défini de contributeurs est de deux et les diverses proportions vont de 95/5 à 50/50 par incréments de 5.

4. Procédé selon la revendication 1 ou la revendication 2, dans lequel le nombre défini de contributeurs est de trois et les diverses proportions vont de 5/5/90 à 30/30/35 par incréments de 5.

5. Procédé selon les revendications 1 à 4, dans lequel l'étape VI du procédé est réalisée par la création d'une statistique de test Chi-carré basée sur la différence résiduelle entre le pourcentage prédit et le pourcentage réel pour chaque allèle, considérant chaque combinaison de paire d'allèles et chaque proportion.

6. Procédé selon les revendications 1 à 5, dans lequel la valeur résiduelle dans l'étape VI comprend des données dans une plage α autour de la valeur résiduelle minimale pour ce marqueur, où α vaut 0,05, donc 5 % autour du minimum, ou 0,1, donc 10 % autour du minimum.

7. Procédé selon les revendications 1 à 6, dans lequel les multiples fois sont au moins 100.

8. Procédé selon les revendications 1 à 7, dans lequel les multiples fois sont au moins 1 000.

9. Procédé selon les revendications 1 à 8, dans lequel les multiples marqueurs courts répétés en tandem sont au moins 10 marqueurs.

10. Procédé pour interroger un mélange d'acides nucléiques dans un échantillon, lequel procédé selon la revendication 1 est effectué successivement ou séquentiellement pour un grand nombre défini de contributeurs pour identifier la proportion la plus vraisemblable d'acides nucléiques dans l'échantillon et ainsi le nombre le plus vraisemblable de contributeurs, par l'identification des combinaisons de paires d'allèles les plus vraisemblables et leur proportion la plus vraisemblable dans l'échantillon pour chaque marqueur, et ainsi des séquences alléliques les plus vraisemblables pour chaque marqueur dans chaque acide nucléique provenant de chaque contributeur.
